# EUROPÄISCHE PATENTANMELDUNG

(11) **EP 0 960 627 A2**
(43) Veröffentlichungstag der Anmeldung: **01.12.1999**
(21) Anmeldenummer: 99109491.3
(22) Anmeldetag: 12.05.1999
(51) Int. Cl.: A61M 5/168, A61M 5/172, A61M 5/142

(54) **Vorrichtung zur zentralen Steuerung und/oder Überwachung von Infusionspumpen**

(30) Priorität: 25.05.1998 DE 19823240
(71) Anmelder: B. BRAUN MELSUNGEN AG, 34212 Melsungen (DE)
(72) Erfinder: Lohmeier, Georg, 82110 Germering (DE); Schmid, Günter, 82110 Germering (DE); Gerstmann, Reinhard, 86916 Kaufering (DE)
(74) Vertreter: Selting, Günther, Dipl.-Ing.

(57) **Zusammenfassung**

Die Erfindung betrifft eine Vorrichtung zur zentralen Steuerung und/oder Überwachung von Infusionspumpen, mit einer Befestigungs- und Trägereinheit, an welcher mehrere hinsichtlich ihrer Funktionen steuerbare und/oder überwachbare Infusionspumpen lösbar anordenbar oder angeordnet sind, und mit einer zentralen Steuer- und/oder Monitoreinheit, mit welcher die Infusionspumpen verbindbar sind, wobei die Befestigungs- und Trägereinheit an vordefinierten Positionen zur Halterung der Infusionspumpen jeweils eine Schnittstelle zur Datenkommunikation für den Anschluß der betreffenden Infusionspumpe aufweist und wobei die zentrale Steuer- und/oder Anzeigeeinheit eine Anzeige aufweist, auf welcher der Zustand der Gesamtheit der mit ihr verbundenen Infusionspumpen darstellbar ist, wobei die Topologie der Darstellung der Infusionspumpen auf der Anzeige der Topologie der Anordnung der Infusionspumpen an der Befestigungs- und Trägereinheit entspricht.

## Beschreibung

Die vorliegende Erfindung bezieht sich auf medizinische Systeme und insbesondere auf Systeme von Infusionspumpen zur Verabreichung von Medikamenten an kritisch kranke Patienten.

In Krankenhäusern und anderen medizinischen Einrichtungen ist es üblich, Medikamente, Vitamine, Nährstoffe und dergleichen an kritisch kranke Patienten mittels sogenannter Infusionspumpen intravenös, d.h. direkt in das Blut, zu verabreichen. Bei Infusionspumpen sind im Wesentlichen zwei Bauformen marktüblich. Zum einen handelt es sich dabei um Spritzenpumpen, die einen Spritzenkolben mit kontrollierter Vorschubgeschwindigkeit bewegen und damit den Spritzeninhalt intravenös verabreichen. Zum anderen sind Pumpen gebräuchlich, die eine auf höherem Niveau in einer Infusionsflasche oder einem Infusionsbeutel befindliche Infusionsflüssigkeit mit kontrollierter Förderrate verabreichen. Beide Bauformen sollen im Folgenden unter dem Begriff Infusionspumpen verstanden werden.

Im modernen klinischen Alltag hat es sich mehr und mehr durchgesetzt, Medikamente nicht mehr manuell mittels Spritze zu verabreichen, sondern voraussehbare Medikamentengaben für Infusionspumpen aufzubereiten und von diesen den Patienten zuführen zu lassen. Diese Zufuhr wird häufig mit konstanter Förderrate gewählt, sie kann jedoch auch diskontinuierlich oder anhand eines Förderprofils mit variabler Förderrate erfolgen. Durch diese häufige Routineanwendung von Infusionspumpen ergibt sich die Situation, daß in einer modernen Intensivstation an einem typischen Intensivpflegeplatz rund ein Dutzend Infusionspumpen installiert sind. Bei der Intensivpflege kritisch kranker Patienten, beispielsweise in der Therapie von Patienten mit Herzerkrankungen, wird nicht selten die Zahl von 20 Infusionspumpen übertroffen.

Nach bisherigem Stand der Technik stellen alle diese Pumpen zusammen mit den anderen um ein Intensivbett angeordneten Medizingeräten ein Konglomerat von Einzelgeräten dar, was, neben vielen anderen Faktoren, die Ergonomie und die Bedienbarkeit von Intensivarbeitsplätzen deutlich erschwert. Dies ist in "medizintechnik, 1/96. S. 7-11" deutlich beschrieben und es wird aus vielerlei Gründen eine Standardisierung und Systematisierung gefordert. Im gleichen Artikel wird auch eine teilweise Lösung für die Handhabung von Infusionspumpen durch das Fluid-System der Firma B. Braun Melsungen AG erwähnt. Dieses geht die Problematik der Arbeitsplatzergonomie und Übersichtlichkeit durch ein Stecksystem für die einzelnen Infusionspumpen innerhalb einer Halteplatte und eine übersichtliche Anzeige der Betriebsparameter jeder einzelnen Infusionspumpe auf einer Anzeigeeinheit zusammen mit weiteren Patientendaten an. Seine Flexibilität erhält das System durch das bedarfsweise Hinzufügen und Entfernen einzelner Infusionspumpen an den dafür vorgesehenen Steckplätzen mit automatischer Herstellung der Energie- und Datenverbindungen.

Eine Möglichkeit der zentralen Eingabe von infusionspumpenbezogenen Parametern ist im US-Patent 4,756,706 beschrieben. Dabei erfolgt die Eingabe dieser Parameter an der zentralen Einheit, welche in stapelartiger Anordnung mechanisch und elektrisch mit den Infusionspumpen verbunden ist und mit diesen Daten austauscht. Ein großer Nachteil dieses Systems besteht in der Eindimensionalität der Anordnung, da die mechanischen und elektrischen Verbindungen von der zentralen Einheit zur ersten Infusionspumpe und dann weiter zur jeweils nächsten Infusionspumpe durch Übereinanderstapeln derselben erfolgen. Die Stapelform erscheint aus praktischen Überlegungen zur Zugänglichkeit jeder einzelnen Infusionspumpe und der zentralen Einheit nur bis zu einer Anzahl von rund zehn Infusionspumpen geeignet. Ein weiterer Nachteil in der praktischen Handhabung ergibt sich aus der stapelartigen Anordnung, da ein Entnehmen einer in der Mitte des Stapels befindlichen Infusionspumpe eine teilweise Demontage des Stapels mit anschließender erneuter Montage bedarf. Dies stellt in der Praxis einen erheblichen Nachteil dar, da, wie weiter unten erläutert wird, ein solches System von Infusionspumpen im klinischen Alltag ein dynamisches System darstellt. Ein dritter bedeutender Nachteil der beschriebenen Ausführungsform liegt darin begründet, daß es sich bei dein System um eine Insellösung handelt, das keinerlei Mittel zur Datenkommunikation mit nicht zum beschriebenen System gehörenden Vorrichtungen gestattet. Des weiteren ist bei diesem System die Verwendung spezieller Infusionspumpen erforderlich, welche das Stapeln zulassen. Die Verwendung vorhandener Pumpen ist normalerweise nicht möglich.

Es gab in der Vergangenheit eine Reihe von Vorschlägen, die einzelne Infusionspumpe mit Mitteln zur Datenkommunikation auszustatten und diese nutzbringend zu verwenden. Stellvertretend sei das US-Patent 5,376,070 erwähnt, das einen Kommunikations-Controller zur Datenkommunikation mit einer einzelnen Infusionspumpe, mit welcher er lösbar zu verbinden ist, umfaßt. Dadurch kann nacheinander jede einzelne Infusionspumpe programmiert werden, um anschließend nach Lösen der Verbindung zum Kommunikations-Controller eigenständig zu arbeiten. Bei diesem System handelt es sich nicht um eine wirkliche zentrale Steuerung und Überwachung, sondern nur um eine komfortablere Möglichkeit der Programmierung jeder Infusionspumpe als an dieser selbst.

Ein weiterer diesbezüglicher Aspekt ist im US-Patent 5,681,285 beschrieben, das aus einer einen elektronischen Speicher enthaltenden Infusionspumpe besteht, wobei der Speicher mit einem Medikamentenverzeichnis geladen werden kann und der Anwender ein Medikament einschließlich diesem zugeordneter Parameter aus diesem Medikamentenverzeichnis auswählen kann. Da die eigentliche Bedienung bei dieser Ausführungsform nach wie vor an der Infusionspumpe selbst vorgenommen wird und nur in relativ großen Zeitabständen eine Verbindung zum extern erstellten Medikamentenverzeichnis hergestellt wird, fehlt hier der zentrale Aspekt weitgehend.

Weitere Anstrengungen wurden in diversen Ausführungsformen unternommen, um Medikamente, welche sich in einer Spritze oder einer Infusionsflasche befinden, maschinenlesbar zu machen. Häufig erfolgte dies mit Hilfe aufgebrachter Strichcodes, die von Mitteln, welche mit der Infusionspumpe in Verbindung stehen oder direkt in sie integriert sind, erfaßt werden können. Als Beispiel hierfür seien die US-Patente 4,978,335 und 5,317,506 genannt. Darüber hinaus finden sich insbesondere in der jüngeren Vergangenheit Beispiele, um Infusionspumpen zusätzlich zu den für die eigentliche Pumpfunktion notwendigen Mitteln, mit Funktionen auszustatten, die eine Erhöhung des Bedienungskomforts durch Implementierung von Zusatzfunktionen bedeuten. Eines dieser Beispiele ist das US-Patent 5,609,575, welches einen Rechner zur Berechnung von Infusionsraten umfaßt, welche aufgrund von Eingaben des Benutzers berechnet werden.

Um den Vorteil eines zentralen Infusionspumpenmonitors zu erkennen, muß man wissen, daß komplexe Infusionspumpen heutzutage beispielsweise über 19 Einzeltasten verfügen können, über welche die vielen Funktionen bedient werden. Einige dieser Tasten sind dabei üblicherweise mit Mehrfachfunktionen belegt. Geht man nur von 10 Infusionspumpen an einem Intensivbett aus, so kommt man auf die Zahl von 190 Einzeltasten für die Bedienung dieses Systems. Hinzu kommt, daß jede Infusionspumpe über eine eigene Anzeige, ein eigenes optisches und akustisches Alarmsystem und eigene Schnittstellen zu übergeordneten Krankenhausdatenerfassungs- und Meldesystemen verfügt. Hinzu kommt, daß die Benutzerschnittstelle einer einzelnen Infusionspumpe derzeit kaum mehr die modernen Anforderungen gemäß ergonomischer Kriterien erfüllt, da der finanzielle Rahmen für die einzelne Infusionspumpe sehr eng ist. Daher finden üblicherweise nur einfache monochrome alphanumerische Flüssigkristallanzeigen oder Sieben-Segment-Leuchtdiodenanzeigen Verwendung. Da andererseits aufgrund der auch bei Infusionspumpen die durch die Steigerung der Rechenleistung möglich gewordene Implementierung immer komplexerer Funktionen sich eine deutliche Diskrepanz zwischen technisch Machbarem und sinnvoll Handhabbarem zeigt, müssen hier neue Wege beschritten werden. Auf einem zentralen Infusionspumpenmonitor läßt sich eine komfortable, zeitgemäße graphische Benutzerschnittstelle installieren, welche die Kommunikation mit dem System erheblich vereinfacht und beschleunigt. Weiterhin ergibt sich durch einen solchen zentralen Infusionspumpenmonitor die Perspektive, materiellen und finanziellen Aufwand bei der einzelnen Infusionspumpe einzusparen, indem nicht zwingend der eigentlichen Pumpenfunktion zugeordnete Funktionselemente ganz oder teilweise aus der Infusionspumpe ausgelagert werden, was letztendlich auch unter Einrechnung des Aufwandes für den zentralen Infusionspumpenmonitor zu einer Reduktion der Kosten des Gesamtsystems führen kann. Als Beispiele solcher nicht der eigentlichen Pumpenfunktion zugeordneter Funktionselemente und damit aus der Infusionspumpe ganz oder teilweise auslagerbar, seien hier die Eingabetasten, die Anzeige oder das Netzteil mit Akkus erwähnt. Ebenfalls unter finanziellen Gesichtspunkten ist die in jüngster Vergangenheit aufgekommene Pflicht zur exakten Abrechnung jeder einzelnen Pflegeleistung an einem Intensivpatienten zu sehen. Derzeit erfolgt eine Dokumentation der durch Infusionspumpen verabreichten Medikamente noch nahezu ausschließlich manuell. Durch einen zentralen Infusionspumpenmonitor vereinfacht sich der Anschluß von Infusionspumpen an ein übergeordnetes Patientendatenmanagementsystem oder an ein Krankenhausinformationssystem wesentlich, da nur noch eine einzige Schnittstelle für den Anschluß einer Vielzahl von Infusionspumpen existiert. Dadurch ergibt sich eine komfortable Möglichkeit, die Medikamentenverabreichung an einen Intensivpatienten für medizinische und für Abrechnungszwecke automatisch zu dokumentieren.

Ein weiterer Anwendungsbereich für eine solche zentrale Steuerung von Infusionspumpen ergibt sich im Bereich der Anästhesie. Hier gibt es seit einigen Jahren Bestrebungen, die bisher in der Regel praktizierte Gasanästhesie durch eine intravenöse Anästhesie zu ersetzen. Gründe hierfür sind unter anderem die immer weiter reduzierten Grenzwerte für die im Operationsraum zulässigen Arbeitsplatzkonzentrationen von Anästhesiegasen, das damit einhergehende Verbot der Beschäftigung Schwangerer im Anästhesiebereich und die geringeren Nebenwirkungen intravenös verabreichter Narkotika. Weitere Gründe für die Anwendung der sogenannten totalintravenösen Anästhesie sind Kostengesichtspunkte, da anstelle eines aufwendigen und teuren Anästhesiegeräts nur ein relativ einfaches und preiswertes Beatmungsgerät und einige gewöhnliche Infusionspumpen nötig sind, sowie Umweltaspekte, da die verwendeten Anästhesiegase allesamt im Verdacht stehen, schädliche Einflüsse auf die Ozonschicht der Erde zu haben.

Für eine derartige totalintravenöse Anästhesie sind mindestens drei Infusionspumpen notwendig, welche die eigentliche Anästhesie, also das Ausschalten des Bewußtseins, die Analgesie, also die Reduktion des Schmerzempfindens und die Muskelrelaxation, also die Entspannung der Muskel herbeiführen. Da die Arbeitsplatzsituation in der Anästhesie derzeit im Wesentlichen von zwei medizintechnischen Geräten geprägt ist, dem Patientenmonitor zur Beobachtung der Vitalparameter EKG, Blutdruck, Sauerstoffsättigung, etc. und dem Narkosegerät zur eigentlichen Anästhesie und Beatmung, der Anästhesist also mit übergeordneten Systemen statt mit Teilsystemen kommuniziert, ist eine Übertragung dieser Arbeitsweise auf die totalintravenöse Anästhesie wünschenswert. Das Ein- und Ausgabegerät für alle am Anästhesiearbeitsplatz installierten Infusionspumpen sollte ein zentraler Infusionspumpenmonitor sein. Dies erleichtert nicht nur die Übersichtlichkeit des Systems, sondern auch die in der Anästhesie gesetzlich vorgeschriebene Dokumentationspflicht, die derzeit noch in vielen Fällen manuell durchgeführt wird.

Im Zusammenhang mit dem anästhesiologischen Arbeitsplatz ist auch das US-Patent 4,741,732 von Bedeutung. In diesem wird eine Vorgehensweise zur Einhaltung eines bestimmten, vom Anwender vorgegebenen, arteriellen Plasmaspiegels eines von einer Infusionspumpe verabreichten Medikaments, z.B. eines Narkotikums, beschrieben. Das Vorgehen beruht auf vorausgegangenen Reihenuntersuchungen an einer hinreichend großen statistisch verwertbaren Probandenzahl, anschließender Modellbildung der physiologischen Vorgänge und anschließender Anwendung des Modells an beliebigen Patienten mit Eingabe von Parametern wie z.B. dein Körpergewicht der Patienten. Beschrieben ist dabei die Einstellung sämtlicher Funktionen direkt an der Infusionspumpe, was diese entsprechend aufwendig werden läßt. Weiterhin ist bei Verwendung von mehr als einem Medikament , dessen Förderrate anhand eines solchen Modells gesteuert wird, die Eingabe der Parameter, wie z.B. des Körpergewichts der Patienten, an jeder einzelnen Infusionspumpe nötig. Auch hier würde ein zentraler Infusionspumpenmonitor Vorteile bedeuten.

Ausgehend von diesem Stand der Technik liegt der Erfindung die Aufgabe zugrunde, eine Vorrichtung zur Vorrichtung zur zentralen Steuerung und/oder Überwachung von Infusionspumpen zu schaffen, welche die zentrale Steuerung/Überwachung mehrere Infusionspumpen ermöglicht, einfach und kostengünstig herstellbar ist und eine ausreichende Flexibilität hinsichtlich der Anzahl und Anordnung der Infusionspumpen aufweist sowie eine hohe Bediensichterheit gewährleistet.

Die Erfindung löst diese Aufgabe mit den Merkmalen des Patentanspruchs 1.

Weitere Ausführungsformen der Erfindung ergeben sich aus den Unteransprüchen.

Die wesentlichen Nachteile der Systeme von Infusionspumpen nach dem gegenwärtigen Stand der Technik werden von der vorliegenden Erfindung vermieden und es ergeben sich eine Reihe von Vorteilen für die Anwendung eines solchen Systems. Die vorliegende Erfindung umfaßt einen zentralen Infusionspumpenmonitor zur Steuerung und Überwachung von zwei oder mehr Infusionspumpen. Der zentrale Infusionspumpenmonitor umfaßt einen oder mehrere Mikroprozessoren mit zugeordneten Programm- und Datenspeichern, sowie eine oder mehrere Anzeige- und Eingabeeinheiten. Weiterhin bestehen eine oder mehrere Datenverbindungen zwischen dein zentralen Infusionspumpenmonitor und den angeschlossenen Infusionspumpen. Der zentrale Infusionspumpenmonitor kann ein Netzteil und eine Batterie für seine eigene Stromversorgung und die Stromversorgung einiger oder aller angeschlossenen Infusionspumpen enthalten. Der zentrale Infusionspumpenmonitor sendet an jede der angeschlossenen Infusionspumpen Steuerbefehle und kann von ihnen Antwortdaten erhalten. Die einzelnen Infusionspumpen sind zu jeder Zeit lösbar an einem Träger montiert, welcher auch die Mittel zur elektrischen Verbindung zwischen den Infusionspumpen und dem zentralen Infusionspumpenmonitor enthält. Der zentrale Infusionspumpenmonitor ist relativ frei plazierbar und nicht an eine bestimmte Position an diesem Träger gebunden, er kann vielmehr auch unabhängig von besagtem Träger z.B. am Patientenbett montiert werden. Das gesamte System ist portabel, so daß es die Patienten bei notwendigen Ortsveränderungen ganz oder teilweise begleiten kann. Der zentrale Infusionspumpenmonitor umfaßt Mittel zur Berechnung der von jeder der angeschlossenen Infusionspumpe zu fördernden Infusionsrate. Für diese Berechnung können vom Anwedner eingegebene patienten- und/oder medikamentenbezogene Parameter, sowie fest im Speicher des zentralen Infusionspumpenmonitors vorhandene Algorithmen herangezogen werden.

Wesentlich ist die visuelle, vorzugsweise graphische Darstellung, der Topologie der angeschlossenen Anordnung von Infusionspumpen auf den Anzeigeeinheiten, die Möglichkeit der Eingabe von Steuerbefehlen und Steuerparametern am zentralen Infusionspumpenmonitor oder in eingeschränkter Form an jeder einzelnen Infusionspumpe und die Anzeige von Betriebsparametern und Werten der angeschlossenen Infusionspumpen auf den Anzeigeeinheiten des zentralen Infusionspumpenmonitors. Ein weiteres Merkmal ist die Anschlußmöglichkeit des zentralen Infusionspumpenmonitors an Dokumentationseinrichtungen, wie z.B. ein Krankenhausinformationssystem, an Ausdruckvorrichtungen, die auch im zentralen Infusionspumpenmonitor integriert sein können und an Meldeeinrichtungen, wie z.B. eine Schwesternrufanlage. Weiterhin kann der Anschluß von Zusatzgeräten möglich sein, die z.B. eine Messung von Flüssigkeitsausscheidungen des Körpers vornehmen, so daß eine Bilanzierung des Flüssigkeitshaushaltes eines Patienten möglich ist. Weitere Anschlußmöglichkeiten umfassen alle aus der Datenverarbeitungstechnik bekannten Geräte, von denen hier nur beispielhaft Massenspeicher; Tastatur, Maus, Barcodeleser- und drucker, Fernbedienung und Geräte zur Datenfernübertragung genannt sind. Alle diese Geräte können sowohl im zentralen Infusionspumpenmonitor eingebaut, als auch extern anschließbar sein.

Gemäß der Idee der Erfindung ist es nicht relevant, ob der zentrale Infusionspumpenmonitor ein eigenes Gerät ist, oder ob dessen Funktionalität in einem anderen Gerät, wie beispielsweise einem Patientenmonitor zur Überwachung der Vitalparameter, einem Beatmungsgerät oder einer bevorzugt anderen Zwecken dienenden Datenverarbeitungseinrichtung integriert ist. Ebenso kann der zentrale Infusionspumpenmonitor innerhalb eines einzigen Gehäuses angeordnet sein oder mit seinen Funktionseinheiten auf mehrere Gehäuse verteilt sein.

Die Erfindung wird nachfolgend anhand in der Zeichnung dargestellter Ausführungsbeispiele näher erläutert. In der Zeichnung zeigen:
- Fig. 1: eine beispielhafte Anordnung eines Systems von Infusionspumpen;
- Fig. 2: in schematischer Form ein beispielhaftes System von Infusionspumpen mit einem zentralen Infusionspumpenmonitor und Anschlüssen zu Peripheriegeräten;
- Fig. 3: ein beispielhaftes Blockdiagramm eines zentralen Infusionspumpenmonitors gemäß vorliegender Erfindung;
- Fig. 4: beispielhaft das Eingabemenü für die Einstellung von Parametern für eine Infusionspumpe;
- Fig. 5: eine beispielhafte Bildschirmanzeige für eine Infusionspumpe innerhalb des Systems von Infusionspumpen und
- Fig. 6: eine beispielhafte Bildschirmanzeige für das System von Infusionspumpen gemäß Fig. 1, die sich aus mehreren Bildschirmanzeigen gemäß Fig. 5 zusammensetzt.

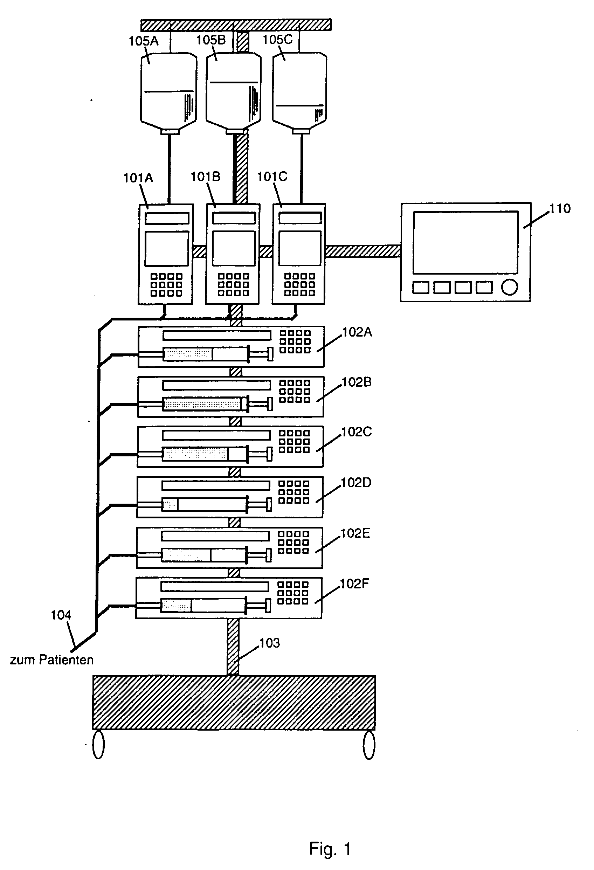

Eine beispielhafte Anordnung eines Systems von Infusionspumpen 101A-101C, 102A-102F mit Flüssigkeitreservoirs 105A-105C und Infusionsleitungen 104 ist in Fig. 1 gezeigt. Dabei handelt es sich um derzeit im klinischen Alltag bereits praktizierte Anordnungen, die je nach medizinischer Notwendigkeit variiert werden können. So könnte bei kritisch kranken Intensivpatienten die Anzahl der Spritzenpumpen 102A-102F erhöht werden oder eine zweite Säule 103 mit Infusionspumpen neben der ersten angeordnet werden. Es ist einsichtig, daß die Anzahl der möglichen Anordnungsvarianten nahezu unbegrenzt ist und im Wesentlichen nur durch die konstruktive Ausführung des mechanischen Trägers für die Infusionspumpen und durch die Anzahl der Infusionspumpen beschränkt ist.

Gemäß eines wesentlichen Aspekts der vorliegenden Erfindung ist die Topologie des Systems von Infusionspumpen, d.h. die räumliche Anordnung der einzelnen Infusionspumpen innerhalb des Systems nicht relevant. Es muß nur sichergestellt sein, daß dein zentralen Infusionspumpenmonitor oder zentralen Steuer- und/oder Monitoreinheit 110 die Topologie des Systems bekannt ist. Dies kann entweder durch eine automatische Erkennung der Topologie oder durch manuelle Eingabe der Topologie oder durch eine Kombination beider Möglichkeiten erfolgen. Da sich die medizinischen Notwendigkeiten bei einem Intensivpatienten im Laufe seines Aufenthalts auf einer Intensivstation ändern, ist ein solches System von Infusionspumpen als ein dynamisches System zu verstehen, bei dem Systemkomponenten im laufenden Betrieb ergänzt oder entfernt werden können und bei dem Betriebsparameter, wie z.B. zu verabreichende Medikamente, zu verabreichende Dosis, zeitlicher Verlauf der Verabreichung und ähnliches im laufenden Betrieb verändert werden können. Aus diesem Grund ist eine automatische Erkennung der Systemtopologie einer manuellen Eingabe derselben wegen des verringerten Bedienungsaufwandes und der reduzierten Fehlermöglichkeiten vorzuziehen. Aus dem selben Grund erfolgt das Hinzufügen bzw. Entfernen einer Systemkomponente bevorzugt derart, daß sowohl die mechanischen als auch die elektrischen Verbindungen zu jeder Systemkomponete ohne weiteres Zutun des Bedieners hergestellt sind bzw. gelöst werden, wenn die jeweilige Systemkomponente korrekt am Träger 103 arretiert bzw. von diesem gelöst worden ist.

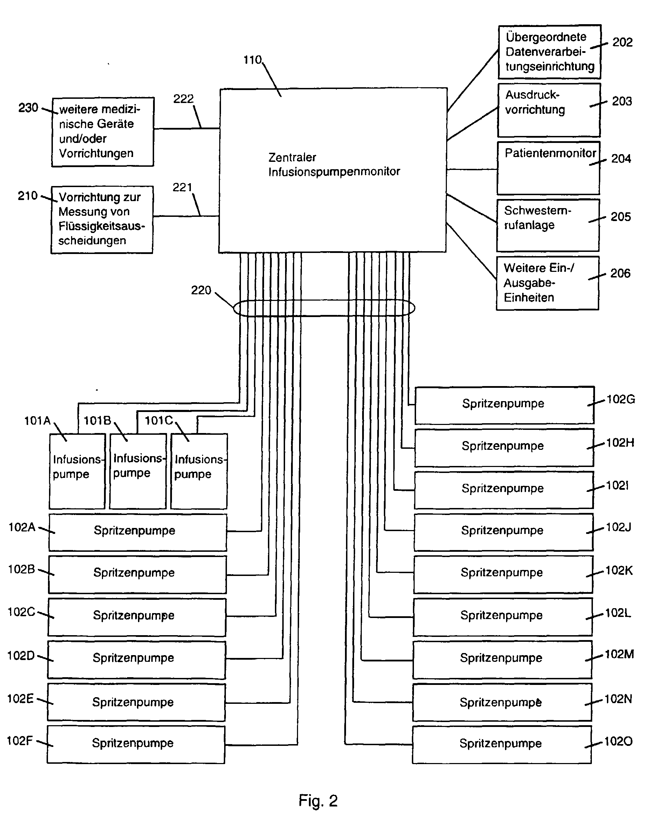

Da die vorliegende Erfindung voll der Verwendung bereits im Markt eingeführter Infusionspumpen einschließlich ihrer Trägersysteme 103 ausgeht und keine Notwendigkeit zur Entwicklung neuer Infusionspumpen und Trägersysteme herleitet, muß die Verbindung jeder einzelnen Infusionspumpe 101A-101C, 102A-102F mit dem zentralen Infusionspumpenmonitor 110 in geeigneter Weise hergestellt werden. Gemäß eines Aspekts der vorliegenden Erfindung erfolgt die Identifikation der Systemtopologie und damit der räumlichen Anordnung jeder einzelnen Infusionspumpe innerhalb des Systems durch die Verdrahtung. Wie in Fig. 2 zu sehen, besteht zwischen jeder einzelnen Infusionspumpe 101A-101C, 102A-102O und dem zentralen Infusionspumpenmonitor 110 eine Datenverbindung 220. Über diese Datenverbindung 220 erhält die einzelne Infusionspumpe 101A-101C, 102A-102O Steuerbefehle vom zentralen Infusionspumpenmonitor 110 und sendet Zustandsinformationen an den zentralen Infusionspumpenmonitor 110. Da es sich bei der vorliegenden Ausführung um eine sternförmige Verdrahtung zwischen dem zentralen Infusionspumpenmonitor 110 und den einzelnen Infusionspumpen 101A-101C, 102A-102O handelt, ist leicht zu erkennen, daß sich die Topologie des Systems aus der Abfrage jeder einzelnen Position durch den zentralen Infusionspumpenmonitor 110 ermitteln läßt. Die einzelne Infusionspumpe 101A-101C, 102A-102O muß lediglich auf ein bestimmtes vom zentralen Infusionspumpenmonitor 110 übermitteltes Kommando ihre Status- und Betriebsdaten übermitteln oder dies kontinuierlich oder zeitdiskret eigenständig veranlassen. Da der klinische Alltag, wie oben beschrieben, eine dynamische Anpaßbarkeit des Systems an medizinische Notwendigkeiten verlangt, muß das Trägersystem 103 einschließlich der Verkabelung innerhalb festgelegter Grenzen erweiterbar sein. Dies kann durch einzelne Elemente erfolgen, die jeweils mehrere Infusionspumpen 101 und/oder 102 mechanisch tragen können und gleichzeitig die korrekte Verkabelung zu jeder einzelnen Infusionspumpe 101 und/oder 102 und zum jeweils nächsten Element und vom jeweils vorhergehenden Element sicherstellen. Es ist auch denkbar, daß die Topologie des Systems die lineare Anordnung verläßt und zweidimensional wird, wie dies beispielhaft in Fig. 2 gezeigt ist. Die beschriebene sternförmige Verbindung der einzelnen Infusionspumpen 101A-101C, 102A-102O mit dem zentralen Infusionspumpenmonitor 110 stellt nur eine mögliche Ausführungsform dar. Es besteht darüber hinaus die Möglichkeit, diese Verbindung gemäß der verschiedenen aus der Datenverarbeitungstechnik hinreichend bekannten Netzwerkanordnungen, wie z.B. ringförmig, busförmig oder aus Mischformen bestehend, zu gestalten. Wesentlich ist nur die eindeutige Identifizierbarkeit jeder einzelnen Infusionspumpe 101A-101C, 102A-102O innerhalb des Systems und deren Lokalisierbarkeit innerhalb der topologischen. Anordnung. Dies ist bei der beschriebenen sternförmigen Anordnung sehr einfach und sicher realisierbar, bei einer anderen Netzwerkanordnung kann dies zusätzliche Aufwendungen in der Software, der Hardware und der konstruktiven Ausführung des Systems bedeuten.

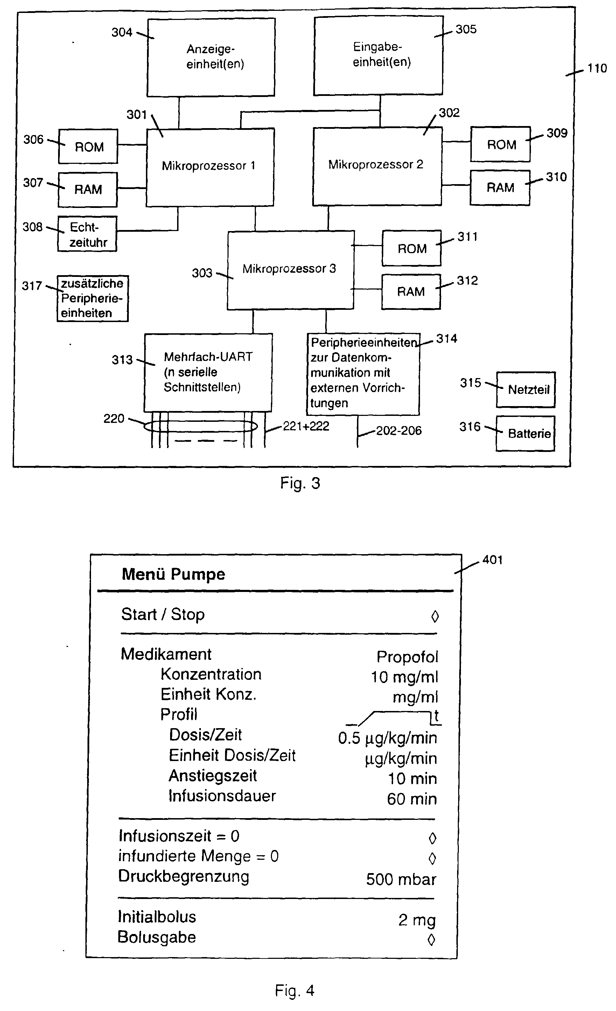

Wenn nun der Benutzer unter Bezugnahme auf Fig. 3 über eine Eingabeeinheit 305 die Einstellung eines Parameters einer der angeschlossenen Infusionspumpen verändert, so erscheint diese Einstellung in einer Anzeigeeinheit 304 des zentralen Infusionspumpenmonitors 110. Nach Bestätigung der Richtigkeit durch den Benutzer wird diese Einstellung von den beiden Mikroprozessoren 301 und 302 unabhängig voneinander erfaßt, verarbeitet und an den Mikroprozessor 303 übertragen. Der Mikroprozessor 303 nimmt einen Vergleich der von den Mikroprozessoren 301 und 302 übertragenen Daten vor und überträgt, im Falle der Übereinstimmung dieser Daten miteinander, die vorn Benutzer gewählte Einstellung mittels einer Datenverbindung 220 an die jeweilige Infusionspumpe. Um die Zuverlässigkeit und die Betriebssicherheit zu erhöhen, antwortet die jeweilige Infusionspumpe mittels einer Datenverbindung 220 mit Antwortdaten, aus denen die Korrektheit der Datenübertragung abgeleitet werden kann. Diese Antwortdaten überträgt die jeweilige Infusionspumpe an den Mikroprozessor 303 und dieser überträgt die Antwortdaten weiter an die Mikroprozessoren 301 und 302 in identischer Form, die unabhängig voneinander einen Vergleich der gesendeten Daten mit den Antwortdaten durchführen, um so die Korrektheit der Datenübertragung zu prüfen. Für zeitabhängige Vorgänge verfügt der zentrale Infusionspumpenmonitor 110 über eine Echtzeituhr 308, die zugleich auch der zweiten Sicherheit gegenüber Fehlern im Arbeitstakt der Mikroprozessoren dient. Jeder der genannten Mikroprozessoren 301-303 verfügt über Programm- 306, 309, 311 und Datenspeicher 307, 310, 312, sowie erforderlichenfalls über zusätzliche Peripherieeinheiten 317. Die Kommunikation zwischen dem zentralen Infusionspumpenmonitor 110 und den einzelnen Infusionspumpen erfolgt gemäß der beispielhaften Ausführung über je eine serielle Datenverbindung 220, die über eine entsprechende Anzahl UARTs Universal Asynchronous Receiver and Transmitter 313 die Verbindung zum Mikroprozessor 303 herstellen. An den zentralen Infusionspumpenmonitor 110 anschließbare Zusatzgeräte 210, 230, wie z.B. zur Messung der Flüssigkeitsausscheidung eines Patienten 210, werden bevorzugt über eine oder mehrere ebenso geartete Datenverbindungen 221, 222 angeschlossen. Als elektrische Spezifikation wurde wegen der hohen Störsicherheit der Datenübetagung die Norm RS485 im Halbduplexverfahren gewählt. Dies stellt jedoch nur eine mögliche Ausführungsform der Datenverbindungen 220-222 dar; weitere Möglichkeiten umfassen alle aus der Datenverarbeitungstechnik bekannten Netzwerktypen, wie z.B. RS232, Ethernet, CAN, 12C, Firewire, USB, etc. Ein weiterer Bestandteil des zentralen Infusionspumpenmonitors ist ein Netzteil 315 und eine wiederaufladbare Batterie 316 zur Stromversorgung des zentralen Infusionspumpenmonitors 110. Zur Datenkommunikation mit externen Vorrichtungen, wie z.B. einer übergeordneten Datenverarbeitungseinrichtung 202, einer Ausdruckvorrichtung 203, einem Patientenmonitor 204, einer Schwesternrufanlage 205 und weiteren Ein-/Ausgabeeinheiten 206, dienen speziell dafür angepaßte Peripheriemittel 314, die mit einem oder mehreren der vorhandenen Mikroprozessoren 301-303 über eine oder mehrere Datenverbindungen gekoppelt sind. Das Netzteil 315 und die Batterie 316 können auch dafür ausgelegt sein, einige oder alle der an den zentralen Infusionspumpenmonitor 110 angeschlossenen Infusionspumpen und Zusatzgeräte mit Strom zu versorgen. In jedem Fall versorgen sie den zentralen Infusionspumpenmonitor 110 mit Strom.

Die Aufteilung in mehrere unabhängige Mikroprozessoren 301-302 ist ein gängiges Verfahren für sicherheitsrelevante Anwendungen, jedoch ist es nicht Voraussetzung im Sinne dieser Erfindung. Weitere Ausführungsformen können alle bekannten Verfahren zur Erzielung einer sogenannten Erstfehlersicherheit bei dem zentralen Infusionspumpenmonitor umfassen. Die Erstfehlersicherheit ist die bei Medizinprodukten gängige Sicherheitsphilosophie, die besagt, daß bei jedem beliebigen ersten Fehler keine Gefahr für die Patienten, die Bediener oder Dritte ausgehen darf. Es ist weiterhin zu fordern, daß ein aufgetretener erster Fehler innerhalb einer Zeitspanne erkannt wird, innerhalb der das Auftreten eines vom ersten Fehler unabhängigen zweiten Fehlers unwahrscheinlich ist. Um diese Erstfehlersicherheit zu erreichen wurde in der vorliegenden Ausführungsform der Erfindung die eigentliche Datenübertragung voll und zu den Infusionspumpen mittels aus der Datenverarbeitungstechnik gängiger Sicherungsverfahren gegen Übertragungsfehler abgesichert. Um Fehler in der Weiterverarbeitung der Benutzereingaben zu Steuerungskommandos an eine einzelne Infusionspumpe zu minimieren, wurde in der vorliegenden Ausführungsform eine hardware- und softwaremäßige Diversität zwischen den Mikroprozessoren 301 und 302 gewählt, d.h. die beiden Mikroprozessoren 301, 302 sind von verschiedenem Typ und auf ihnen laufen verschiedene Programme, die ihre Ergebnisse auf verschiedenen Rechenwegen erzielen. Da bestimmte Teile des zentralen Infusionspumpenmonitors nicht oder nur mit unverhältnismäßig hohem Aufwand erstfehlersicher zu machen sind, wie z.B. die Anzeigeeinheiten 304, wurde hierfür eine Zweistufigkeit der Benutzereingaben gewählt, um die gleiche Sicherheit zu erreichen. In einem ersten Schritt gibt der Benutzer über ein Eingabemittel 305 seine Befehle ein. In der gewählten Ausführungsform handelt es sich um eine kombinierte Eingabe über Tasten und Drehrad. Diese Eingaben erscheinen dann an mindestens einer Anzeigeeinheit 304. In der gewählten Ausführungsform handelt es sich um eine menügeführte graphische Benutzeroberfläche auf einer farbigen Aktivmatrix-Flüssigkristallanzeige, ähnlich der aus dem Datenverarbeitungsbereich hinlänglich bekannten graphischen Benutzeroberflächen. Nach Abschluß aller Eingaben innerhalb eines Menüs erscheinen nochmals alle sicherheitsrelevanten Eingaben an anderer Bildschirmposition, so daß der Benutzer einen Vergleich zwischen den von ihm eingegebenen Befehlen und den vom zentralen Infusionspumpenmonitor 110 erkannten Befehlen durchführen kann. Erst nach positiver Bestätigung der Richtigkeit durch den Benutzer erfolgt die Verarbeitung der Befehle und letztlich die Übermittlung der Befehle an eine der Infusionspumpen. In Fig. 4 ist ein solches Eingabemenü 401 beispielhaft dargestellt.

Nach erfolgreicher Übermittelung der Befehle an eine Infusionspumpe kann diese je nach ihrer Ausführungsform mehr oder weniger autark weiterarbeiten, auch wenn sie vom zentralen Infusionspumpenmonitor 110 durch Entnahme aus dem Träger 103 abgekoppelt wird. Bei vollständig autark arbeitenden Infusionspumpen läuft eine Abfolge von Befehlen in der Infusionspumpe ab, welche die gleichen Aktionen hervorrufen, als wenn die Infusionspumpe mit dem zentralen Infusionspumenmonitor 110 verbunden wäre. Bei nicht vollständig autark arbeitenden Infusionspumpen, die mit weniger Funktionsumfang ausgestattet sind, würde bei Entnahme der Infusionspumpe aus dein Träger 103 die zuletzt eingestellte Förderrate beibehalten werden bzw. in Sonderfällen wie z.B. den weiter unten beschriebenen Profilen würde die Infusionspumpe bei Entnahme aus dem Träger 103 in den sicheren Zustand übergehen, was in der Regel der Pumpen-Stop ist.

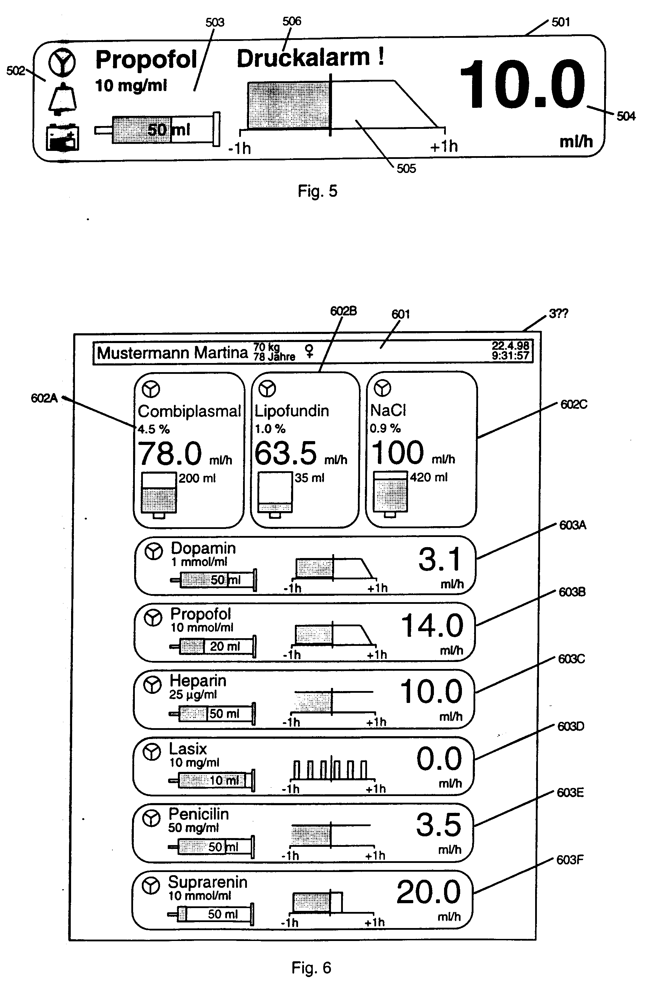

Gemäß eines weiteren Aspekts der Erfindung werden alle angeschlossenen Infusionspumpen an mindestens einer Anzeigeeinheit 304 des zentralen Infusionspumpenmonitors 110 vorzugsweise in graphischer Form angezeigt. Um das Risiko einer Fehlbedienung auszuschalten, erfolgt die Anzeige schematisiert so, daß sich auf der betreffenden Anzeigeeinheit 304 eine Repräsentation des topologischen Aufbaus des realen Systems von Infusionspumpen zeigt. So ergibt sich für das Bedienpersonal eine einfache Zuordnung jeder Infusionspumpe zu ihrer Anzeige auf dein zentralen Infusionspumpenmonitor 110. Dies stellt eine wichtige Eigenschaft im Hinblick auf die Systemsicherheit und Akzeptanz eines solchen Systems dar. Die Erteilung eines Befehls an eine bestimmte Infusionspumpe erfolgt nun durch Auswahl der gewünschten Infusionspumpe mit Hilfe einer Eingabeeinheit 305. Diese Auswahl erfolgt durch Mittel, welche bei graphischen Benutzeroberflächen gängig sind, wie z.B. Umrahmen, Ändern der Hintergrundfarbe, Invertieren eines Bildschirmbereichs oder sonstiges Hervorheben des ausgewählten Objekts. Nach positiver Bestätigung der Auswahl durch den Benutzer erhält der Benutzer Zugang zu einem Menü 401, beispielsweise gemäß Fig. 4, mit allen Einstellmöglichkeiten für eine bestimmte Infusionspumpe. Diese können sein: Medikamentenname, Konzentration des Medikaments, gewünschte Dosierung, gewünschtes Förderprofil, usw. Die Einstellung erfolgt in der bevorzugten Ausführungsform durch Anwahl eines Menüpunktes mittels geeigneter Eingabemittel 305, wie z.B. eines Drehknopfs, der einen Bildschirmcursor zur Bewegung veranlaßt, und anschließendes Bestätigen der Wahl mittels geeigneter Eingabemittel 305, z.B. durch Druck auf den Drehknopf. Die Einstellung eines gewünschten Einstellwertes, z.B. der Konzentration, erfolgt nunmehr durch Drehen des Drehknopfs bis der gewünschte Wert erreicht ist und anschließenden Druck auf den Drehknopf zur Bestätigung der Einstellung. Einige Bestandteile dieses Menüs 401 können variabel gestaltet sein, wie dies beispielsweise für die Eingabe der Parameter eines Profils sinnvoll ist. Das in Fig. 4 gezeigte Menü 401 kann den gesamten Anzeigebereich oder nur einen Teil des Anzeigebereichs der jeweiligen Anzeigeeinheit 304 bedecken. In der beispielhaften Ausführungsform belegt dieses Menü nur einen Teil des Anzeigebereichs. Ein zweiter Teil zeigt alle Betriebsdaten der ausgewählten Infusionspumpe z.B. in einer Darstellung gemäß Fig. 5. Ein dritter Teil kann die weiter oben beschriebene Anzeige aller sicherheitsrelevanten Einstellungen für eine nochmalige-Bestätigung durch den Benutzer zeigen. Durch die graphische Repräsentation des topologischen Aufbaus des Systems von Infusionspumpen erhält der Benutzer auch mit einem Blick einen Überblick über den Zustand aller angeschlossenen Infusionspumpen. Dieser Zustand kann den aktuellen Betriebszustand laufend/gestoppt, den aktuellen Füllstand des Flüssigkeitsreservoirs wie z.B. der Spritze, die aktuelle Förderrate, den Batteriezustand und ähnliches erfassen. Ein besonderer Vorteil des zentralen Infusionspumpenmonitors liegt in der Zentralisierung der Alarmabgabe. Durch die beschriebene graphische Repräsentation des topologischen Systemaufbaus läßt sich eine alarmgebende Infusionspumpe schnell und sicher lokalisieren, indem sie auf den Anzeigeeinheiten deutlich von den übrigen Infusionspumpen abgehoben erscheint. Darüber hinaus können der Alarmgrund und weitere Informationen auf den Anzeigeeinheiten eingeblendet werden. Im Zusammenhang mit der Alarmabgabe. wird ein weiterer Vorteil des beschriebenen zentralen Infusionspumpenmonitors 8110 deutlich, der in dein deutlich verringerten Aufwand zum Anschluß an eine sogenannte Schwesternrufanlage 205 liegt. Dabei handelt es sich um in Krankenhäusern übliche Meldeanlagen, die im Falle eines Alarms eines angeschlossenen Geräts ein optisches und/oder akustisches Signal am Platz des Pflegepersonals auslösen. Bei Patientenmonitoren zur Überwachung der Vitalparameter ist diese Technik neben der zentralen Darstellung der Vitalparameter seit langem etabliert; bei Infusionspumpen unterblieb bisher meist die Anbindung an eine Schwestenrufanlage 205 wegen der relativ hohen Zahl der Einzelgeräte pro Intensivbett. Durch den zentralen Infusionspumpenmonitor 110 reduziert sich der Anschlußaufwand auf einen einzigen Anschluß für alle im System befindlichen Infusionspumpen, was letztlich einen finanziellen Vorteil bedeutet.

Ein besonderer Vorteil der graphischen Benutzerschnittstelle zeigt sich in der komfortablen Möglichkeit sogenannte Profile einzustellen und zu visualisieren. Bei einem Profil handelt es sich um die, einer Funktion der Zeit und möglicher weiterer Parameter folgende, Förderrate einer Infusionspumpe. Im einfachsten Fall eines Profils fördert die Infusionspumpe eine zeitlich konstante Förderrate. Weitere Profile können mit An- und Abstiegsrampen versehen, mit einer zeitlichen oder einer volumenmäßigen Begrenzung versehen, einer beliebigen mathematischen Funktion wie z.B. der Exponentialfunktion folgend und ähnliches sein. Eine weitere Ausführungsform eines Profils ist die im medizinischen Alltag häufig vorkommende Bolusgabe in bestimmten zeitlichen Abständen, z.B. 3 mal täglich eine bestimmte Menge. Derartige Profile können am zentralen Infusionspumpenmonitor 110 wesentlich komfortabler ausgewählt und in ihren Parametern eingestellt werden, als es an der einzelnen Infusionspumpe mangels entsprechender Visualisierungsmittel denkbar wäre, was bisher in der Praxis dazu führte, daß derartige Profile praktisch ausschließlich durch Aktionen des Pflegepersonals ausgeführt wurden. Die automatische Ausführung von Profilen und deren Umsetzung in Steuerbefehle für die einzelne Infusionspumpe entlastet eindeutig das Pflegepersonal und führt zu einer exakteren Dosierung der einzelnen Medikamente. Als eine Erweiterung der Profile ist die Möglichkeit des sogenannten teach-in zu sehen. Beim teach-in-Betrieb speichert der zentrale Infusionspumpenmonitor alle Aktionen des Bedieners, d.h. der Bediener steuert die Infusionspumpe wie bisher von Hand und benutzt für seine Entscheidungen sein komplexes medizinisches Wissen, das größtenteils nicht in Formeln oder Regeln zu fassen ist. Nach Beendigung des teach-in-Betriebs kann der Bediener dieser gespeicherten Abfolge von Aktionen eine eindeutige Kennung zuweisen, unter der zukünftig diese Abfolge von Aktionen jederzeit wieder ohne weiteres Zutun des Bedieners reproduzierbar ist. Im Bereich der Automatisierungstechnik ist dieses Prinzip des teach-in seit langem gebräuchlich; im Zusammenhang mit der totalintravenösen Anästhesie verspricht diese Vorgehensweise auch im medizinischen Bereich Bedeutung zu erlangen, da die Dosierung der Narkotika, wie schon bei der konventionellen Gasanästhesie üblich, von Parametern wie Körpergewicht der Patienten, Alter und Geschlecht, sowie von ärztlicher Erfahrung abhängt. Als eine weitere Erweiterung der Profile können pharmakokinetische Modelle betrachtet werden, bei denen die Verteilung eines verabreichten Medikamentes im Körper des Patienten durch geeignete Modelle simuliert wird. Diese Modelle sind medikamentenspezifisch und erhalten weitere Eingabeparameter wie Patientengewicht oder -alter. Die Zielgröße stellt dabei ein gewünschter Plasmaspiegel im Blut der Patienten dar, also eine gewünschte Konzentration dieses Medikamentes. Diese Ziel-Konzentration kann zeitlich konstant sein, was nur den Ersatz der vom Körper pro Zeiteinheit resorbierten Menge des Medikaments bedingt, oder sie kann zeitvariabel sein. Aufgabe des pharmakokinetischen Modells ist es nun, durch einen Regelalgorithmus die Förderrate der Infusionspumpe so zu steuern, daß, unter Berücksichtigung der Eingabeparameter, die tatsächlich im Blut vorhandene Konzentration dieses Medikamentes gleich der Ziel-Konzentration ist. Ein solches pharmakokinetisches Modell kann sowohl in der Infusionspumpe selbst, als auch im zentralen Infusionspumpenmonitor 110 implementiert sein, wobei aus den vorstehenden Erläuterungen zur Bedienerfreundlichkeit dem zentralen Infusionspumpenmonitor 110 der Vorzug zu geben ist.

In direktem Zusammenhang mit den genannten Profilen steht ein weiterer Aspekt der Erfindung. Er besteht darin, die bisher vom Arzt meist manuell in einem Verordnungsbogen eingetragenen Medikamentenverordnungen und dann vom Pflegepersonal im Lauf eines Tages verabreichten Medikamentengaben, zumindest was die über Infusionpumpen zugeführten Medikamente betrifft, zu automatisieren. Hierzu verfügt der zentrale Infusionspumpenmonitor 8110 über mindestens eine Schnittstelle zu mindestens einer übergeordneten Datenverarbeitungseinrichtung 202, in welche der Verordnungsplan anstelle des manuell auszufüllenden Verordnungsbogens eingegeben wird. Eine andere Möglichkeit ist die Eingabe des Verordnungsplans in einen Patientenmonitor 204, wozu der zentrale Infusionspumpenmonitor eine Datenverbindung haben kann. Eine dritte Möglichkeit ist die Eingabe des Verordnungsplans in den zentralen Infusionspumpenmonitor 110 selbst. Über welche der beschriebenen Möglichkeiten der Verordnungsplan auch eingegeben wurde, er gelangt in den zentralen Infusionspumpenmonitor 110 und wird dort in der Art ausgeführt, wie ihn eine Pflegekraft manuell ausführen würde, d.h. zu den vom Arzt im Verordnungsplan definierten Zeiten werden Pumpen automatisch gestartet oder gestoppt oder es werden Förderraten geändert und dergleichen. Dies ist ein weiterer Schritt in der Entlastung des Pflegepersonals voll Routineaufgaben und reduziert darüber hinaus die Fehlermöglichkeiten durch Verringerung der Komplexität des Ablaufs um eine Ebene.

Die in einer Intensivstation verwendeten Medikamente entstammen in der Regel einem bestimmten Bestand an Medikamenten, die je nach medizinischer Erfordernis an Patienten angewandt werden. Da jedes Krankenhaus über einen eigenen Bestand an Medikamenten verfügt, liegt es nahe, diese nicht bei jeder Anwendung neu einzugeben, sondern in einer Medikamentendatenbank zu speichern und sie im Bedarfsfall daraus auszuwählen. Da es für die eindeutige Dokumentation unabdingbar ist, daß der zentrale Infusionspumpenmonitor 110 über die Daten des von jeder Infusionspumpe verabreichten Medikamentes verfügt, ist in der vorliegenden Erfindung eine Kopplung des zentralen Infusionspumpenmonitors 110 mit einer Medikamentendatenbank vorgesehen. Diese Medikamentendatenbank kann, wie bereits oben beim Verordnungsplan beschrieben, sowohl im zentralen Infusionspumpenmonitor 110, als auch in externen Einrichtungen z.B. 202 angeordnet sein. Diese Medikamentendatenbank wird in der Regel einmalig gemäß den Erfordernissen des jeweiligen Krankenhauses erstellt und bei Bedarf gepflegt. Sie enthält für jedes Medikament Angaben wie z.B. Medikamentenname, zulässige Einheiten der Konzentration, zulässiger Bereich der Förderrate, Dosierparameter und ähnliches. Die Eingabe in einem Bildschirmmenü wie in Fig. 4 gezeigt, beschränkt sich dann auf die Auswahl des Medikamentennamens aus der Medikamentendatenbank, der Eingabe der Konzentration, sowie in der Eingabe der Förderparameter, wie z.B. Auswahl eines Profils. Als ein Attribut zum jeweiligen Medikament kann die Medikamentendatenbank für das jeweilige Medikament zulässige Profile enthalten. Weiterhin kann gemäß eines wichtigen Aspekts der Erfindung jedem Medikament eine bestimmte Farbe zugeordnet werden. Diese bestimmte Farbe findet dann in der Bildschirmanzeige gemäß Fig. 5 ihre Repräsentation in farbigen Anzeigen aller einem Medikament zugeordneten Eigenschaften, wie z.B. der Medikamentenname, die Konzentration, die aktuelle Förderrate, die noch im Flüssigkeitsreservoir befindliche Menge, der Verlauf des Profils in der Vergangenheit und der Zukunft. Durch diese Möglichkeit der selektiven Farbgebung läßt sich die Übersichtlichkeit des Systems deutlich steigern. So ist es z.B. möglich, allen herzwirksamen Medikamenten die Farbe rot zuzuordnen, was bisher häufig durch farbige Spritzenaufkleber geschah. Wie weiterhin in Fig. 5 gezeigt, ist die Bildschirmanzeige 501 jeder Infusionspumpe in verschiedene Bereiche unterteilt. Ein erster Bereich 502 zeigt dabei aktuelle Zustandsanzeigen der Pumpe, wie z.B. Pumpe läuft/Pumpe steht dargestellt durch ein sich scheinbar drehendes Rad, Netz- oder Batteriebetrieb dargestellt durch das entsprechende Normsymbol, Alarme dargestellt durch eine Glocke und dergleichen. Ein zweiter Bereich 503 zeigt die Bezeichnung des von der Infusionspumpe verabreichten Medikaments, seine Konzentration und die noch in der Spritze befindliche Flüssigkeitsrestmenge in Relation zum gesamten Spritzeninhalt. Im Falle einer Infusionspumpe, welche eine Flüssigkeit aus einem auf höherem Nivaeu befindlichen Flüssigkeitsreservoir fördert, wird statt der Restmenge die bereits infundierte Menge angezeigt. Ein dritter Bereich 504 zeigt die aktuelle Förderrate der Infusionspumpe mit physikalischer Einheit. Ein vierter Bereich 505 zeigt das Profil gemäß welchem die Infusionspumpe fördert. Dieses Profil wird bevorzugt als graphische Darstellung gezeigt, mit der Zeitachse in der Horizontalen und der Förderrate in der Vertikalen. Dabei zeigt der Bereich links von der Mitte die Vergangenheit und der Bereich rechts von der Mitte die Zukunft.

Der Vergangenheitsbereich wird als ausgefüllte Fläche dargestellt, der Zunkunftsbereich als Linie entsprechend der gewählten Förderrate. Die Mittellinie stellt den aktuellen Zeitpunkt dar. Um klinische Gegebenheiten flexibel abzudecken, ist die Skalierung der Zeitachse, also der für Vergangenheit und Zukunft gezeigte Zeitraum, vom Anwender einstellbar; so kann beispielsweise in Intensivstationen ein Zeitraum voll 6 Stunden vor und zurück eingestellt werden, während für Operationsraumanwendungen kürzere Zeiträume von z.B. 30 Minuten vor und zurück gewählt werden. In einem fünften Bereich 506 werden Alarme und Meldungen, welche die jeweilige Infusionspumpe betreffen angezeigt.

Weiterhin erlaubt der zentrale Infusionspumpenmonitor den Aufruf und die Darstellung weiterer Menüs, die wie das weiter oben beschriebene Menü 401 aus Fig. 4 mit Hilfe einer Eingabeeinheit 305 bedient werden. Ein derartiges Menü ist beispielsweise ein Patienten-Menü zur Eingabe aller patientenbezogenen Daten. Ein weiteres derartiges Menü ist ein Trend-Menü zur übersichtlichen Darstellung der Medikamentenverabreichung über der Zeit durch jede an das System angeschlossene Infusionspumpe. Ein weiteres derartiges Menü ist ein allgemeines Einstell-Menü zur Eingabe allgemeiner Daten wie des Datums und der Uhrzeit, sowie weiterer Einstellparameter für die internen Funktionen des zentralen Infusionspumpenmonitors 110. Diese Menüs sind hier nicht bildlich gezeigt, da sie sich nicht prinzipiell von entsprechenden Menüs unterscheiden, wie sie beispielsweise in Patientenmonitoren nach dein derzeitigen Stand der Technik zu finden sind.

Für jede angeschlossene Infusionspumpe existiert eine solche Anzeige 501, wie sie beispielhaft in Fig. 5 gezeigt ist. Werden nun mehrere solcher Anzeigen gemäß Fig. 5 derart kombiniert, daß sich an einer Anzeigeeinheit 304 die Topologie des Systems von Infusionspumpen ablesen läßt, ergibt sich eine Darstellung wie in Fig. 6. Hier ist beispielhaft die Bildschirmanzeige für das System von Infusionspumpen 101A-101C, 102A-102F aus Fig. 1 gezeigt. Die einzelnen Infusionspumpen 101A-101C, 102A-102F besitzen dabei jeweils ihnen zugeordnete Anzeigefelder 602A-602C, 603A-603F, Zusätzlich zu den Feldern der einzelnen Infusionspumpen gibt es auch einen oder mehrere Bereiche 601 auf mindestens einer Anzeigeeinheit 304, der allgemeine, nicht einer bestimmten Infusionspumpe zuzuordnende Anzeigen, wie z.B. Name, Alter und Gewicht der Patienten, Datum und Uhrzeit und dergleichen enthält. Die einzelnen Felder 602A-602C, 603A-603F für die einzelnen Infusionspumpen können auch von der Standarddarstellung abgewandelt sein, wenn es die Gegebenheiten erfordern. So ist beispielsweise in Fig. 6 zu sehen, daß Infusionspumpen mit Infusionsbeutel 101A-101C eine etwas andere Darstellung als Spritzenpumpen 102A-102F haben können. Ebenso ist es denkbar, verschiedene Typen von Infusionspumpen unterschiedlich darzustellen. Wesentlich bleibt der schnelle und einfache Überblick über das System von Infusionspumpen durch die graphische Repräsentation der topologischen Anordnung der einzelnen Infusionspumpen auf mindestens einer Anzeigeeinheit des zentralen Infusionspumpenmonitors 110.

## Patentansprüche

1. Vorrichtung zur zentralen Steuerung und/oder Überwachung von Infusionspumpen,
a) mit einer Befestigungs- und Trägereinheit, an welcher mehrere hinsichtlich ihrer Funktionen steuerbare und/oder überwachbare Infusionspumpen lösbar anordenbar oder angeordnet sind, und
b) mit einer zentralen Steuer- und/oder Monitoreinheit, mit welcher die Infusionspumpen verbindbar sind,
**dadurch gekennzeichnet**,
c) daß die Befestigungs- und Trägereinheit an vordefinierten Positionen zur Halterung der Infusionspumpen jeweils eine Schnittstelle zur Datenkommunikation für den Anschluß der betreffenden Infusionspumpe aufweist und
d) daß die zentrale Steuer- und/oder Anzeigeeinheit eine Anzeige aufweist, auf welcher der Zustand der Gesamtheit der mit ihr verbundenen Infusionspumpen darstellbar ist, wobei die Topologie der Darstellung der Infusionspumpen auf der Anzeige der Topologie der Anordnung der Infusionspumpen an der Befestigungs- und Trägereinheit entspricht.

2. Vorrichtung nach Anspruch 1, **dadurch gekennzeichnet**, daß zusätzlich zu den Schnittstellen für die Datenkommunikation an einer oder mehreren der vordefinierten Positionen für die Infusionspumpen eine Schnittstelle zur Energieversorgung der betreffenden Infusionspumpe vorgesehen ist

3. Vorrichtung nach Anspruch 2, **dadurch gekennzeichnet**, daß an der Befestigungs- und Trägereinheit eine zentrale Energieversorgungseinheit vorgesehen ist, welche mit den Schnittstellen zur Energieversorgung verbunden ist.

4. Vorrichtung nach Anspruch 3, **dadurch gekennzeichnet**, daß die Infusionspumpen und die zentrale Steuer- und/oder Monitoreinheit zusätzlich über eine autonome Not-Energieversorgung verfügen, welche bei einem Ausfall der zentralen Energieversorgungseinheit die sichere Funktion der Infusionspumpen und der Steuer- und/oder Monitoreinheit gewährleisten.

5. Vorrichtung nach einem der vorhergehenden Ansprüche, **dadurch gekennzeichnet**, daß die zentrale Steuer- und oder Monitoreinheit eine an der Befestigungs- und Trägereinheit vorgesehene Untereinheit und eine damit verbundene übergeordnete Einheit aufweist, wobei die Untereinheit selbständig vorgegebene Funktionen der Infusionspumpen steuert und/oder die Kommunikation der Infusionspumpen mit der übergeordneten Einheit steuert.

6. Vorrichtung nach Anspruch 5, **dadurch gekennzeichnet**, daß die übergeordnete Einheit in einem separaten Gehäuse vorgesehen ist.

7. Vorrichtung nach einem der vorhergehenden Ansprüche, **dadurch gekennzeichnet**, daß die Steuer- und/oder Monitoreinheit oder die übergeordnete Einheit lösbar mit der Befestigung- und Trägereinheit verbunden und frei plazierbar ausgebildet ist.

8. Vorrichtung nach einem der vorhergehenden Ansprüche, **dadurch gekennzeichnet**, daß die Befestigung der Infusionspumpen an dem Befestigungs- und Trägersystem derart ausgebildet ist, daß bei einem mechanischen Befestigen gleichzeitig eine Verbindung mit der betreffenden Schnittstelle zur Datenkommunikation mit der zentralen Steuer- und/oder Monitoreinheit herstellbar ist.

9. Vorrichtung nach einem der vorhergehenden Ansprüche, **dadurch gekennzeichnet**, daß die Schnittstellen zur Datenkommunikation und die Infusionspumpen so ausgebildet sind, daß nach einem Lösen einer Infusionspumpe von dem Befestigungs- und Trägersystem die Infusionspumpe das Lösen umgehend detektiert und in seiner vorbestimmten Funktion autark weiterarbeitet oder den zuletzt innegehabten Betriebszustand beibehält oder in einen sicheren Zustand übergeht.

10. Vorrichtung nach einem der vorhergehenden Ansprüche, **dadurch gekennzeichnet**, daß zusätzlich zu den Infusionspumpen ein oder mehrere weitere Module an der Befestigungs- und Trägereinheit anordenbar und/oder mit der zentralen Steuer- und/oder Monitoreinheit verbindbar sind, welche physiologische Parameter eines Patienten erfassen.

11. Vorrichtung nach einem der vorhergehenden Ansprüche, **dadurch gekennzeichnet**, daß die zentrale Steuer- und/oder Monitoreinheit, vorzugsweise die untergeordnete Einheit, mit einer oder mehreren anderen Vorrichtungen innerhalb der Patientenumgebung oder einer anderen relativ nichttransportierbaren Vorrichtung innerhalb der medizinischen Einrichtung zum Datenaustausch verbindbar ist.

12. Vorrichtung nach einem der vorhergehenden Ansprüche, **dadurch gekennzeichnet**, daß die zentrale Steuer- und/oder Monitoreinheit, vorzugsweise die übergeordnete Einheit Eingabemittel umfaßt, welche die Eingabe von Parametern durch den Anwender zur Steuerung der Infusionspumpen ermöglichen.

13. Vorrichtung nach Anspruch 12, **dadurch gekennzeichnet**, daß das System Mittel umfaßt, um Infusionsraten aus vorn Anwender eingegebenen Parametern zu berechnen, wobei die Parameter vorzugsweise die Konzentration eines physiologisch wirksamen Stoffes innerhalb der Flüssigkeit und/oder das Körpergewicht des Patienten und/oder eine gewünschte Konzentration des phsyiologisch wirksamen Stoffes im Blut eines Patienten umfassen.

14. Vorrichtung nach einem der Ansprüche 10 bis 13, **dadurch gekennzeichnet**, daß ein oder mehrere, von einer oder mehreren geeigneten Meßvorrichtungen gemessene physiologische Parameter oder von diesen gemessenen physiologischen Parametern abgeleitete Parameter das Ergebnis der Berechnungen der Infusionsraten beeinflussen.

15. Vorrichtung nach einem der vorhergehenden Ansprüche, **dadurch gekennzeichnet**, daß der zentralen Steuer- und/oder Monitoreinheit manuell oder von einer weiteren Vorrichtung ein Verzeichnis von Medikamenten und mit diesen Medikamenten in Bezug stehende Parameter eingebbar und speicherbar sind.

16. Vorrichtung nach Anspruch 15, **dadurch gekennzeichnet**, daß dieses Verzeichnis von Medikamenten einschließlich der mit diesen Medikamenten in Bezug stehenden Parametern durch manuelle Eingaben veränderbar ist.

17. Vorrichtung nach einem der vorhergehenden Ansprüche, **dadurch gekennzeichnet**, daß besondere Zustände der einzelnen Infusionspumpen, insbesondere Alarme, mittels der zentralen Steuer- und/oder Monitoreinheit anzeigbar sind.
